# EUROPEAN PATENT SPECIFICATION

(11) **EP 4 460 695 B1**
(45) Date of publication and mention of the grant of the patent: **24.12.2025**
(21) Application number: 23700486.6
(22) Date of filing: 09.01.2023
(51) Int. Cl.: G01N 33/487, G01N 35/00

(54) **SEMI-AUTOMATED PATCH-CLAMP APPARATUS AND A METHOD FOR CARRYING OUT A PATCH-CLAMP PROCEDURE**
HALBAUTOMATISCHES PATCH-CLAMP-GERÄT UND VERFAHREN ZUR DURCHFÜHRUNG EINES PATCH-CLAMP-VERFAHRENS
APPAREIL PATCH-CLAMP SEMI-AUTOMATISÉ ET PROCÉDÉ PERMETTANT DE METTRE EN OEUVRE UNE PROCÉDURE PATCH-CLAMP

(30) Priority: 07.01.2022 EP 22150580
(43) Date of publication of application: 13.11.2024
(73) Proprietor: Sophion Bioscience A/S, 2750 Ballerup (DK)
(72) Inventor: HENNEKE, Jens, 2750 Ballerup (DK); SKAFTE-PEDERSEN, Peder, 2750 Ballerup (DK); JIN, Wenxi, 2750 Ballerup (DK); JUHL, Patrick Martin, 2750 Ballerup (DK); HOMANN, Lasse, 2750 Ballerup (DK); JACOBSEN, Rasmus Bjørn, 2750 Ballerup (DK)
(74) Representative: Inspicos P/S
(86) International application number: PCT/EP2023/050288
(87) International publication number: WO 2023/131698

(56) References cited:
- EP-A2- 3 730 925
- WO-A1-2018/213357
- US-A1- 2011 045 582
- US-A1- 2017 138 926

## Description

### TECHNICAL FIELD

The invention relates to a patch-clamp apparatus comprising a patch-clamp plate manipulator configured to receive a patch-clamp plate comprising a plurality of patch-clamp sites. The patch-clamp apparatus comprises a computerised controller. The invention further relates to a method of carrying out a patch-clamp procedure.

### BACKGROUND

Ion channels are membrane proteins in cells of living organisms. A flow of ions into and out of the cells is controlled by the membrane, and the ion channels are important targets for a variety of drugs.

Ion channels are commonly studied with a technique called patch-clamping. The cells are applied to a patch-clamp plate comprising a plurality of patch-clamp sites.

The on-cell, whole-cell, or perforated patch configurations of patch-clamp are widely accepted as providing the best methods of measuring ion channel activity for drug screening. In these methods, ion currents flowing through ion channels are measured directly and with high resolution by sensitive current amplifiers.

A protocol specifies a pressure, a potential, or a current which is applied to the patch-clamp site, and a current, a capacitance, or a cell potential etc. is subsequently measured across the ion channel.

Users need efficient methods to investigate ion channels. While a high throughput in the screening process is important, flexibility and freedom for the user to dynamically interact with the investigation process is highly desired.

The general idea of electrically insulating a patch of membrane and studying the ion channels in that patch under voltage-clamp conditions is outlined in Neher, Sakmann, and Steinback (1978) "The Extracellular Patch-clamp, A Method For Resolving Currents Through Individual Open Channels In Biological Membranes", Pflüger Arch. 375; 219-278. Recent developments in patch-clamp methodology have seen the introduction of planar substrates (e.g a silicon chip) in place of conventional glass micro pipette (for example, see WO 01/25769 and Mayer, 2000). Additional background art includes US 8,268,260 and US 2017/138926.

Unfortunately, current patch-clamp methods and devices suffer from shortcomings. Particularly, existing methods are either manual methods or fully automatic methods. The manual methods require highly skilled operators performing time consuming testing, and throughput is typically insufficient for industrial testing. The automatic methods are carried out by large and expensive machines with very little user interaction once an experimental protocol has started. While offering a high throughput, they do not offer the opportunity to dynamically amend ongoing experiments, and each experiment must be planned from start to end before it is initiated.

### SUMMARY

It is an object of embodiments of the invention to improve patch-clamping and particularly to improve the balance between throughput and dynamic interaction during testing. It is a further object to effectively bridge a gap between manual and automated measurements while reducing operator error and to allow a more robust, reproducible measurement known from automated measurements while retaining the flexibility of manual patch experiments demanded by expert users.

For this and other purposes, the invention, in a first aspect, provides a semi-automatic apparatus wherein the controller is configured to:
- define a patch-clamp procedure comprising a plurality of process steps, wherein each process step is assigned to automatic execution by the manipulator or manual execution by a user,
- provide instructions for a user to carry out manual process steps assigned to manual execution, and
- provide commands for the manipulator regarding automatic process steps assigned to automatic execution.

The controller initiates the process steps assigned to automatic execution by dispatching the commands to the manipulator and initiates the manual process steps assigned to manual execution by dispatching the instructions to the user.

Since the controller has the patch-clamp experiment as a defined procedure with a plurality of steps, and each step is assigned to either manual or automatic execution, the controller may change between manual and automatic processing during a patch-clamp procedure. This provides improved throughput during automatic processing and preserves the ability to dynamically interact during the experiment.

When used herein, "semi-automated" means that the apparatus is configured to allow certain process steps to be carried out manually while allowing automatic execution of other process steps.

When used herein "patch-clamp procedure" means a selection of process steps and a specific order they are carried out.

When used herein "process steps" means single activities included in the patch-clamp procedure and they could be defined e.g. in a protocol.

When used herein "protocol" means a pre-specified electrical signal, pressure signal, optical signal, or combinations thereof which can be applied to the patch-clamp site by the manipulator.

The patch-clamp plate manipulator is configured to carry out all necessary handling of the patch-clamp plate. The manipulator comprises mechanical components including inlets facilitating supply of solutions to the patch-clamp sites and for controlling pressure at the patch-clamp sites. Additionally, the manipulator comprises electronic components for carrying out a procedure where an electrical and/or a pressure signal and/or an optical signal is applied to the patch-clamp site and an electrical signal is received from the patch-clamp site.

The computerised controller is configured with a user interface for communication with the user and with a data interface to the manipulator such that the controller can control the manipulator and thus the automatic process steps. Additionally, the controller may have other interfaces, e.g. for communicating data with external computers.

The patch-clamp procedure comprises a plurality of pre-defined process steps. These process steps are defined in the memory of the controller, and they include manual process steps to be executed manually by the user and automatic process steps for automatic execution by the manipulator. The controller is programmed to provide instructions both relative to the manual process steps and relative to the automatic process steps.

The instructions for the user are typically provided on a computer screen, e.g. in written form, and typically with graphical interaction illustrating e.g. where to add solutions etc. The interface may also feed results back to the user during execution of the procedure and thereby allow the user to amend subsequent steps in view of the results obtained by previous steps. The controller may be configured to communicate with the user not only by the screen, but also by audio signals or combinations of optical signals and audible signals.

The controller may particularly comprise a plurality of patch-clamp procedures. The user may select a predefined patch-clamp procedure from a procedure library and thereby have several predefined process steps loaded into the controller and ready to be carried out.

The controller may also allow the user to define new patch-clamp procedures. These may be defined e.g. via the computer screen or they may be uploaded from another computer system.

For defining new patch-clamp procedures, the controller may comprise a plurality of predefined process steps. Each process step may be predefined either as an automatic process step or a manual process step.

The controller may comprise a plurality of patch-clamp protocols. Each protocol defines a sequence of signals applied automatically by the manipulator to the patch-clamp sites. The protocols may be included in a protocol library from which the user can select a protocol for a specific process step. At least one of the protocols may specify at least one of a pressure, a potential, or a current applied by the manipulator to the patch-clamp site.

Additionally, the controller may allow the user to define and optionally to store new protocols when needed. These may be defined e.g. via the computer screen or they may be uploaded from another computer system.

The protocols may, in addition to pressure and electrical signal values, specify an optic signal to flash the cell, e.g. by light of different wave-lengths, e.g. ultra-violet light e.g. to provide light-evoked currents recorded under voltage clamp.

The controller may comprise measurement steps defining how readings are made automatically by the manipulator at the patch-clamp sites. At least one of the measurement steps may specify reading of an electrical signal or an optical signal from the patch-clamp site.

The controller may also allow the user to define new measurement steps. These may be defined e.g. via the computer screen or they may be uploaded from another computer system.

The controller may allow the user to select predefined protocols and/or predefined measurement steps for a patch-clamp procedure, and particularly to allow such selection while the patch-clamp procedure is carried out. This allows the user to change the patch-clamp procedure depending on results obtained during the procedure.

The controller may be configured to assign the manual process steps to a time critical group of manual process steps or to a non-time critical group of manual process steps.

The instructions for a user to carry out steps assigned to manual execution may be provided with a timer countdown triggering automatic shifting to a subsequent manual process step or automatic process step execution when the manual process step is assigned in the time critical group of manual process steps. This allows the controller to determine the timing between two subsequent process steps and thereby ensures that certain timing parameters are observed.

A user triggered acknowledgement may trigger a subsequent manual process step or automatic process step execution when the manual process step is assigned in the non-time critical group of manual process steps. This allows the user to determine the timing when the timing is less critical for the experiment being carried out. The user triggered acknowledgement could be pushing of a button on the touch screen, or voice activated acknowledgement, e.g. by the user giving a "next" command.

The controller may be configured to allow the user to re-assign a manual process step to an automatic process step, or to re-assign an automatic process step to a manual process step. This may e.g. by done during the execution of a patch-clamp procedure.

The controller may be configured with a user interface allowing the user to store a patch-clamp procedure after having amended the patch-clamp procedure, and optionally to add meta-data related to a patch-clamp procedure. The controller may define a data file comprising the meta data with data related to the patch-clamp procedure, and it may allow such meta-data to be exported to another computer system. The user interface may allow the user to add the meta data individually to selected process steps or to the patch-clamp procedure as such. In one example, the user may e.g. make a comment to a result obtained in a measurement step, or the user may make a comment about the entire procedure.

Additionally, or alternatively, the controller may automatically add meta data related to the patch-clamp procedure and/or to individual process steps, e.g. data and time stamps, or results obtained in measurement steps.

The controller may be configured to communicate to the user which process step is being executed, e.g. by visualising graphically on the screen the current step, the previous step and the next step or by visualising that the current step is step nr. x out of a total number of y steps.

The controller may be configured to allow the user to skip a process step and jump to a subsequent process step and to communicate results of a process step to the user before initiating a subsequent process step.

The controller may comprise a graphical user interface and the instructions for a user to carry out manual process steps may comprise graphical illustrations related to the patch-clamp plate or the plurality of patch-clamp sites, e.g. illustrating specifically in which inlet to add a particular solution etc.

The controller comprises a predefined sequence in which the process steps are executed. The sequence may e.g. determine that one process step is carried out after another process step and before yet another process step. The sequence may also determine that two process steps are carried out simultaneously after another process step and before yet another process step. The controller may comprise a user interface allowing the user to change the sequence, e.g. by keying in a different sequence, or by graphically drag-and-dropping a step to a different location in a sequence of steps. A change in the sequence may be added either automatically or manually to said meta data.

In a second aspect, the invention provides a method of carrying out a patch-clamp procedure, the method comprising predefining the procedure in the form of a plurality of manual process steps and a plurality of automatic process steps in a computer system and allowing the computer system to trigger execution of the automatic process steps by electronically controlling an actuator and to trigger the manual process steps by instructing a user.

The method may include any step implicit in view of the apparatus according to the first aspect of the invention.

Further details of the technology are provided in the enclosed dependent claims, figures, and examples.

### LEGENDS TO THE FIGURES

The technology is illustrated by means of the following schematic illustrations, in which:
Fig, 1 illustrates an apparatus according to the invention;
Fig. 2 illustrates a patch-clamp procedure schematically;
Fig. 3 illustrates steps of a patch-clamp procedure;
Fig. 4 illustrates user amendments of a patch-clamp procedure, and
Figs. 5-10 illustrate different user interfaces of the controller.

### DETAILED DISCLOSURE

The patch-clamp technique is used to study ionic currents in individual isolated living cells, tissue sections, or patches of cell membrane. The technique is inter alia used in studies of neurons, cardiomyocytes, muscle fibres, and pancreatic beta cells.

Different patch-clamping techniques are used. In voltage clamp technique, the voltage across the cell membrane is controlled, and the resulting currents are recorded. In current clamp technique, the current passing across the membrane is controlled and the resulting changes in voltage are recorded.

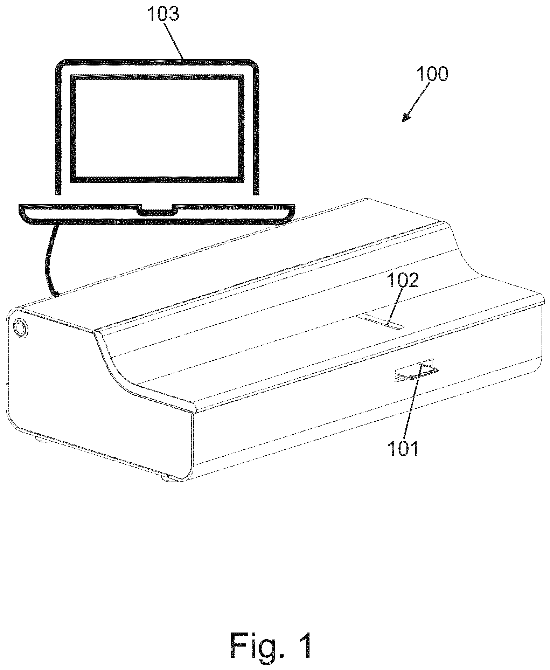

Fig. 1 illustrates a semi-automated patch-clamp apparatus (100). The apparatus comprises a slot 101 for receiving a patch-clamp plate. The patch-clamp plate captures and holds cells while patch-clamp procedure is carried out.

The patch-clamp plate is of a kind used in automated patch-clamp (APC) measurements for high-throughput recordings of ion channel currents in living cells. Such patch-clamp plates are assembled from several components with a range of properties that are advantageous for APC measurements.

The patch-clamp plate is typically "single-use". Accordingly, they are easily replaced by removing a used, plate from the slot 101, and inserting a new single-use plate in the slot. In this manner, contamination between experiments can be reduced or eliminated.

The patch-clamp plate comprises a plurality of intracellular (IC) inlets arranged in at least one array. Each IC inlet is in fluid connection with an IC chamber. The patch-clamp plate also comprises a plurality of extracellular (EC) inlets arranged in another array. Each EC inlet is in fluid connection with an EC chamber.

The patch-clamp plate also comprises a patch-clamp chip arranged between each EC chamber and each IC chamber, each patch-clamp chip comprising one or more patch holes. The at least one patch hole extends through the patch-clamp chip and provides a fluid connection between EC and said IC chambers. The biological cells to be tested are captured on the patch hole during patch-clamp experiments.

Each IC chamber of the patch-clamp plate comprises an IC electrode; and each EC chamber comprises an EC electrode.

The patch-clamp plate may additionally comprise a cover which shields the testing environment and provides inlets for liquid access to the experiment site. A flexible gasket may also be present, to form the liquid- and electrical seal between the EC and IC chambers.

At the experiment site, a tight, giga-ohm (GΩ) seal is established between the cell membrane and patch hole using physiological solutions. This allows a user to avoid "seal-enhancing" ions, such as fluoride, which are often required in APC experiments and have been reported to modulate ion channel function

The patch-clamp plate may contain multiple measurement sites depending on the required throughput. Each "measurement site" comprises the IC chamber, EC chamber and at least one patch-clamp chip, with associated EC/IC inlets and outlets.

The microfluidic channel on the extracellular side allows rapid liquid exchange with multiple solution additions to the same cell during an experiment. The solution consumption is significantly reduced as solution exchange can be achieved with less than 10µL of solution

The apparatus comprises an array of inlets 102 for liquid addition e.g. by use of manual pipetting. Any number of inlets may be provided. In the disclosed apparatus, there is an array of 8 inlets. The array contains 8 inlets configured for receiving 8 separate pipets.

The apparatus comprises a patch-clamp plate manipulator receiving the patch-clamp plate and configured to carry out automatic procedures. The manipulator comprises mechanically moving components for bringing the 8 separate inlets in communication with the EC and/or IC chambers of the plate. Subsequently, the 8 different solutions can be treated individually.

The manipulator also comprises required electronic components for applying a signal to the patch-clamp site and for reading a signal from the patch-clamp site. The applied signal includes an electrical signal, a pressure signal, and optionally, an optic signal, e.g. a flash of light of a certain wave-length.

The apparatus comprises a connection for pressurised gas, e.g. air, vacuum, and optionally a connector for water or for communication of other consumables for the experiment.

The apparatus comprises a computerised controller configured with a user interface in the form of a computer screen 103, in this case a touch screen allowing two-way communication between the controller and the user.

The controller is implemented in a CPU with memory and computer executable code for enabling various functions which will be described in further details below. The illustrated apparatus comprises a dedicated controller specifically made for carrying out the patch-clamp procedure. The controller could, alternatively, be constituted at least partly by a standard computer system, e.g. a PC. The controller comprises a data interface for communication of data externally, e.g. for exporting results and for importing patch-clamp procedures, process steps, and/or protocols.

Those skilled in the art will appreciate that the functions of the patch clamp device may be implemented using standard hardware circuits, using software programs and data in conjunction with a suitably programmed digital microprocessor or general-purpose computer, and/or using applications specific integrated circuitry, and/or using one or more digital signal processors. Software program instructions and data may be stored on a non-transitory, computer-readable storage medium, and when the instructions are executed by a computer or other suitable processor control, the computer or processor performs the functions associated with those instructions.

Devices known in the art are made for fully automatic procedures where the entire procedure is prespecified in the memory of the controller. This is efficient but results in reduced flexibility and very little interaction with the user. The controller of the apparatus illustrated in Fig. 1 is configured to define a patch-clamp procedure comprising a plurality of process steps to be executed one after the other.

Some of the process steps are for manual process execution by the user and some of the process steps are to be carried out automatically by the apparatus.

The controller provides instructions for the user relative to the manual process steps, and it controls the manipulator relative to automatic execution of the automatic process steps.

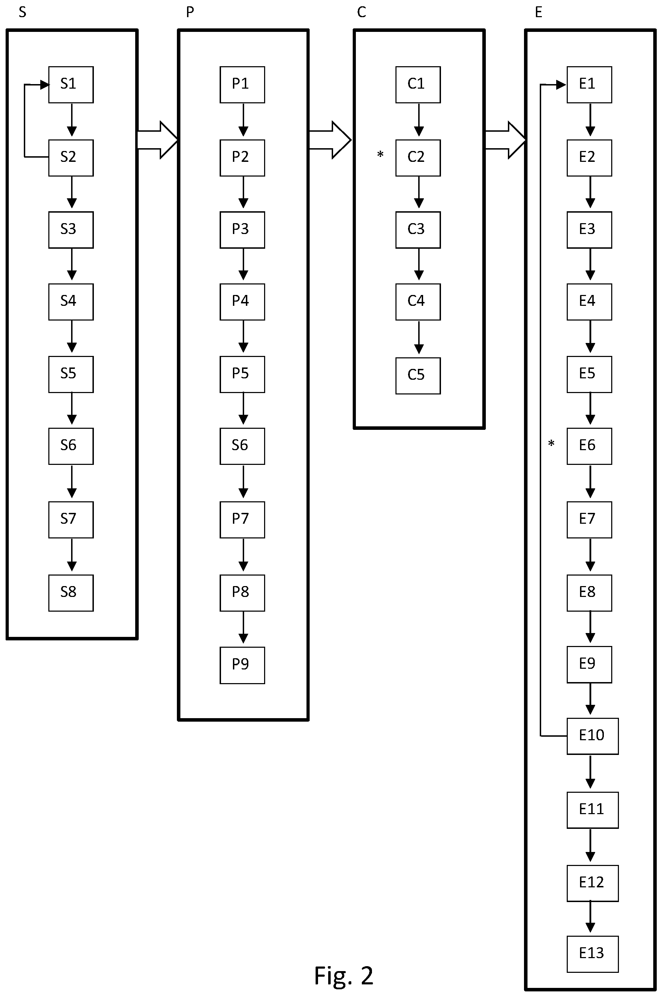

Fig. 2 illustrates schematically a workflow with a plurality of process steps illustrating an example of a patch-clamp procedure defined in the memory of the controller.

The patch-clamp procedure comprises a plurality of process steps. In this implementation of the invention, the process steps are grouped in four different groups of process steps.

A first group of process steps is referred to as "set-up" or simply group S and contains 8 process steps in which an experiment is being setup. The number of these process steps begin with an S, i.e. S1-S8.

A second group of process steps is referred to as "Priming of patch-clamp plate" or simply group P and contains 9 process steps in which the patch-clamp plate is being primed. The number of these process steps begin with a P, i.e. P1-P9.

The priming process may include a priming protocol by which complete evacuation of air from the microfluidic channels before the beginning of the experiment is carried out.

Cells are applied via an extracellular channel and positioned at the patch hole(s) by low, negative pressure across the patch hole(s). When properly sealed, a brief suction pulse brings the cell into the whole-cell configuration, where it is held in place by low, negative pressure throughout subsequent patch clamp experiments. The extracellular solution can be exchanged throughout the experiment allowing precise addition and washout of solutions.

A third group of process steps is referred to as "Cell positioning and Whole Cell (WC) formation" or simply group C and contains 5 process steps in which the cell is positioned, and WC is formed. The number of these process steps begin with a C, i.e. C1-C5.

A fourth group of process steps is an optional, free definable set of process steps. This could be a proprietary standard used by a certain user. Herein, this is referred to as "E-phys experiment" or simply group E and contains 12 process steps in which the E-phys experiment is carried out. The number of these process steps begin with an E, i.e. E1-E12.

The process steps are outlined below:
S-process steps:
   S1. [M] User must insert patch-clamp plate
   S2. [A] Barcode is scanned
   S3. [M] User must Choose no. of sites (1-8)
   S4. [M] User must enter metadata (cell type, solution names etc)
   S5. [M] Optional: User can preload list of solutions to test in the e-phys experiment (name+concentration)
   S6. [M] User must select a protocol from a library to be used for WC formation
   S7. [M] Optional: User can select a fixed sequence of steps and protocols to run in the e-phys experiment
   S8. [M] User must press start
P-process steps:
   P1. [A] Manipulator prepares patch-clamp plate for addition of IC liquid
   P2. [M] User is instructed to add IC liquid
   P3. [M] User adds liquid and presses "ok"
   P4. [A] Manipulator seals plate and the controller executes a protocol of applied pressure steps to prime the plate with the IC solution
   P5. [A] Manipulator prepares patch-clamp plate for addition of EC liquid
   P6. [M] User is instructed to add EC solution
   P7. [M] User adds liquid and presses "ok"
   P8. [A] Manipulator seals plate and the controller executes a protocol of applied pressure steps to prime the plate with EC solution
   P9. [A] Controller measures individually on 1-8 sites the electrical characteristics of the patch-clamp plate primed with IC and EC solution. If characteristics are within user-defined limits each site will be cleared for further use.
C-process steps:
   C1. [M] User is prompted to add a volume of cell solution. A countdown timer instructs the user when to add cells in one site at a time.
   C2. [M] User adds the cell solution.
   C3. [A] In a timely manner the controller starts a protocol of applied pressures to position a cell to the patch hole in each of 1-8 sites. The controller measures an electrical readout to determine when a cell is positioned.
   C4. [A] The controller starts a protocol of applied pressures and/or electrical potential to put the cell into whole-cell formation. The controller measures an electrical readout to determine when a cell is in whole-cell formation.
   C5. [A] 0-8 sites are ready for e-phys experiments.
E-process steps:
   E1. [M] User choose a site to execute a protocol
   E2. [M] User selects a solution to test
   E3. [M] User selects a protocol from a library
   E4. [M] User press "execute"
   E5. [A] A countdown timer instructs the user to add the selected solution
   E6. [M] The user adds the selected solution
   E7. [A] In a timely manner the controller executes the selected protocol
   E8. [A] The controller collects data and relevant processed data is presented to the user.
   E9. [A] Based on the processed data the user can select how to proceed with the experiment
   E10. [A] The steps 1-8 are repeated for up to 8 sites depending on the user preference.
   E11. [M] When the user acknowledges being finished testing solutions the e-phys experiment can be ended
   E12. [A] Manipulator releases plate so the user can remove it
   E13. [A] Data is subsequently exported.

Each step includes an indication [A] for automatic or [M] for manual. This is a definition comprised in the controller and distinguishing manual process steps from automatic process steps. During execution of a patch-clamp procedure, the identification of automatic and manual process steps allows the controller to execute the patch-clamp procedure by dispatching the commands to the manipulator for execution of the automatic process steps and by dispatching the instructions to the user for manual execution of the manual process steps.

In Step S1, the corresponding instruction dispatched by the controller via the touch screen is "please insert a new patch-clamp plate".

In Step S2, the corresponding command to the manipulator is to scan the barcode on the patch-clamp plate. This is an electronic command submitted to a bar code reader included in the patch-clamp plate manipulator, and in response to receiving this instruction, the bar code reader reads a bar code on the patch-clamp plate and returns a data string representing the read value.

In Step S3, the corresponding instruction dispatched by the controller via the touch screen is "please select a number of available sites"

In Step S4, the corresponding instruction dispatched by the controller via the touch screen is "please add meta-data", or the instructions are guiding the user to add specific data. In an example, the user may benefit from pull-down menus where a patch-clamp procedure ID, date, user-ID and other kinds of predefined meta-data can be selected and associated with the patch-clamp procedure which is being initiated.

In Step S5, the corresponding instruction dispatched by the controller via the touch screen is "please select solutions" and the user is guided by pull-down menus where different solutions and concentrations can be selected and associated with the patch-clamp procedure which is being initiated.

In Step S6, the corresponding instruction dispatched by the controller via the touch screen is "please select a protocol" and the user is guided by pull-down menus where different protocols from a library of protocols can be selected and associated with the patch-clamp procedure which is being initiated.

In Step S7, the corresponding instruction dispatched by the controller via the touch screen is "please select a sequence" and the user is guided by a graphical user interface illustrating a pre-defined sequence of steps and protocols for the patch-clamp procedure, and the user can change the predefined sequence.

In Step S8, the corresponding instruction dispatched by the controller via the touch screen is "please start the patch-clamp procedure by pressing start" and the user has the option to wait until being ready. At this point, additional menus may allow the user to abort the procedure, and/or to store the procedure for execution at a later point in time.

Fig. 2 illustrates the procedure schematically. The arrows indicate that the user can scroll between each step. Between the groups of steps, the user can only move forward, i.e. from group S to P to C and to E. In an alternative implementation, the user may go in both directions, e.g. back to Setup after having executed steps in the Priming group.

Additionally, the user can cancel steps, or insert additional steps between existing steps.

In Fig. 2, some of the process steps are marked with a star. This indicates that the process step is a time critical manual process step, and in this case, the controller will strictly follow a time schedule and automatically continue to the next process step when a specifically recorded time variable is exceeded, e.g. after 10 seconds.

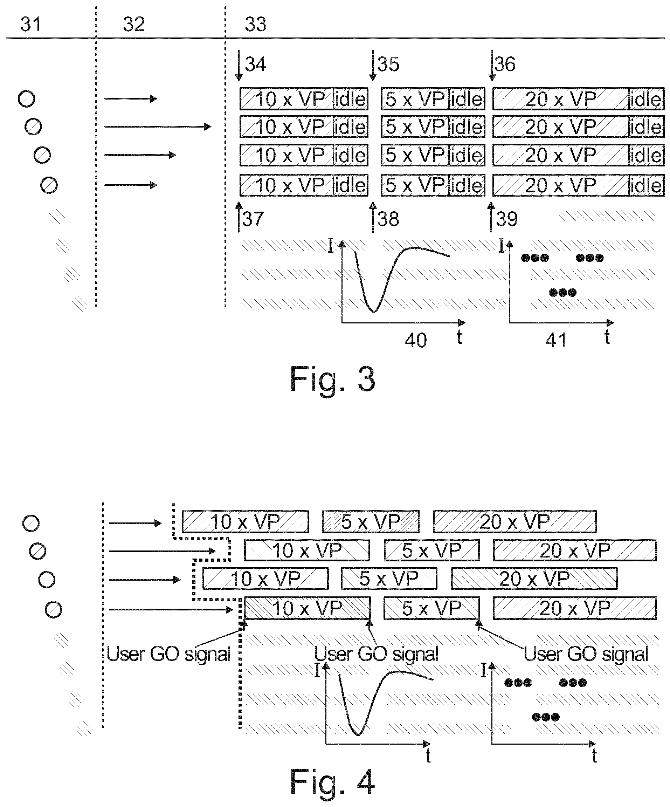

Figs. 3 and 4 illustrate user interaction relative to steps in groups P, C and E.

Fig. 3 illustrates a table with three columns 31, 32 and 33. Column 31 comprises a selection of 4 out of 8 possible sites, i.e. the patch-clamp procedure is reduced to the use of four of the 8 sites. In column 32, it is indicated that the adaptive WC period has a duration which is individual for each of the 4 sites. The duration is indicated by the corresponding arrow. In column 33, a predefined selection of protocols is illustrated. In this example, the protocols are identically chosen for the four different sites. The arrows 34, 35, 36 indicate a manual task for the user to add liquid to the four sites, and the arrows 37, 38, 39 indicate a manual task for the user to acknowledge that procedure to continue to the next step of the predefined patch-clamping procedure. The output 40 and 41 are provided to the user during the execution of the patch-clamp procedure and allows the user to obtain knowledge which can be used to amend an ongoing procedure, while it is executed.

Fig. 4 illustrates a different user interaction where the user, during the execution of the patch-clamp procedure, has amended the protocols with respect to length, nature, and timing. This could e.g. be based on output 40, 41 obtained during the procedure and can be individually amended across the selected sites.

The present invention has been described with reference to several embodiments and figures. However, the skilled person can select and combine various embodiments within the scope of the invention, which is defined by the appended claims.

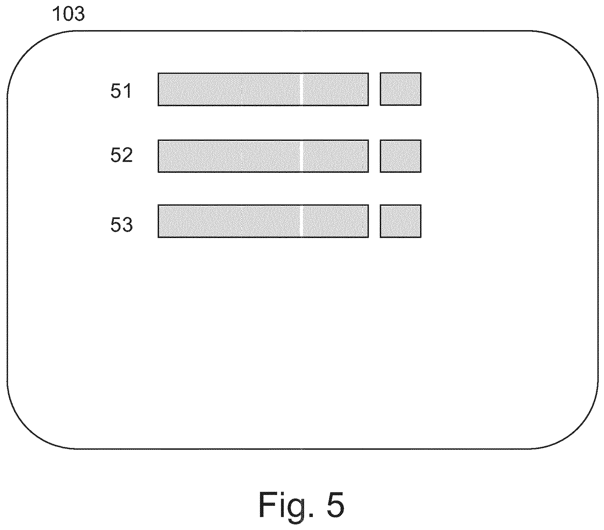

The controller comprises protocols each defining a sequence of signals applied automatically by the manipulator to the patch-clamp sites. Additionally, the controller comprises measurement steps defining readings made automatically by the manipulator at the patch claim sites. Fig. 5 illustrates a user interface presented on the computer screen 103 and allowing the user to define the protocols and/or measurement steps or to amend predefined protocols and/or predefined measurement steps, or to select predefined protocols and/or predefined measurement steps for a patch-clamp procedure. In this user interface, the procedure is selected in a scroll down menu in window 51, the protocol is selected in a scroll down menu 52 and the measurement steps are selected in a scroll down menu 53. The selected protocols and measurement steps are automatically added to the selected patch-clamp procedure according to a standard implemented in the controller. Subsequently, the user can amend not only the protocols and measurement steps but also the specific order of the process steps and thereby the order in which the protocols are executed, and the measurements are carried out.

The user interface illustrated in Fig. 5 can be activated through the entire patch-clamp procedure and thereby allows the user to define and/or select the protocols and/or the measurement steps during execution of a patch-clamp procedure.

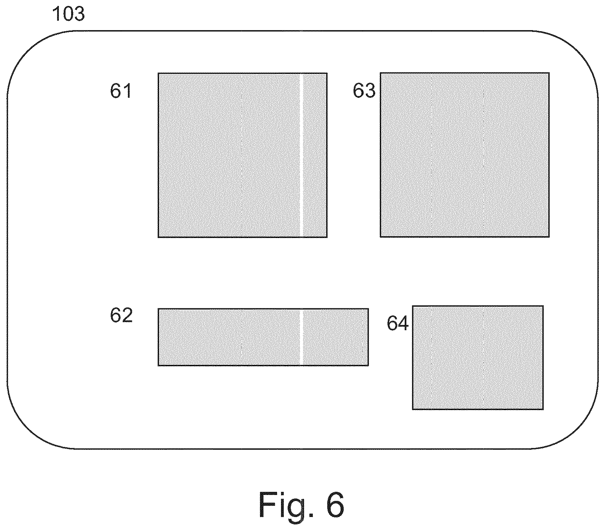

Fig. 6 illustrates a user interface presented on the computer screen 103 and by which the controller communicates the measurements, i.e. electrical signals, pressure signals, or optical signals measured at the patch-clamp site, to the user. The user interface comprises four windows 61, 62, 63, and 64 each assigned to present a specific reading. Since particularly readings are always presented in specifically assigned windows, reading is safe, fast, and easy. The controller allows the user to reprogram each window to match a personal preference of the user. Other numbers of windows may be defined depending on the need for feedback of results to the user during a patch-clamp procedure.

The user interface illustrated in Fig. 6 can be activated throughout the patch-clamp procedure and thereby allows the user to gain access to the results while the procedure is carried out.

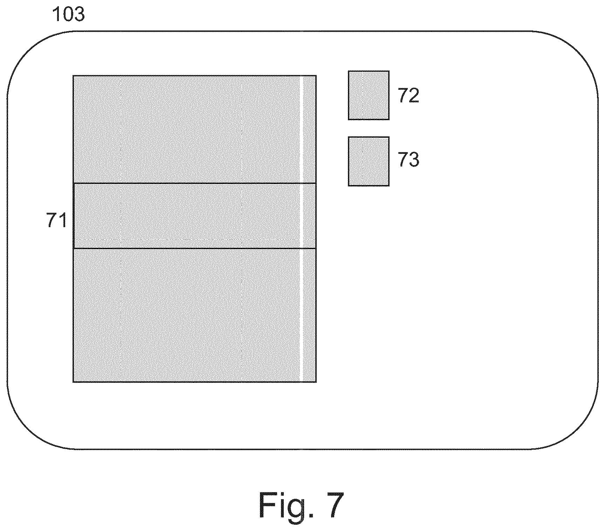

Fig. 7 illustrates a user interface presented on the computer screen 103. In this user interface, the user can re-assign a manual process step to an automatic process step, or to re-assign an automatic process step to a manual process step. In the window 71, the user may select each process step from a pull-down menu, and by clicking button 72 or 73, the user may assign the process step to automatic execution or manual execution. The controller is configured to present an inactive state by graying-out impossible selections thereby preventing the user to assign a process step to manual execution, when only automatic execution is possible, vice versa.

All user interfaces allowing the user to define or amend a patch-clamp procedure, a process step, a protocol, or a measurement step is complemented with a save-selection button by which the user can instruct the controller to store the amendment either by overwriting the previous patch-clamp procedure, process step, protocol, or measurement step or by creating a new patch-clamp procedure, process step, protocol, or measurement step.

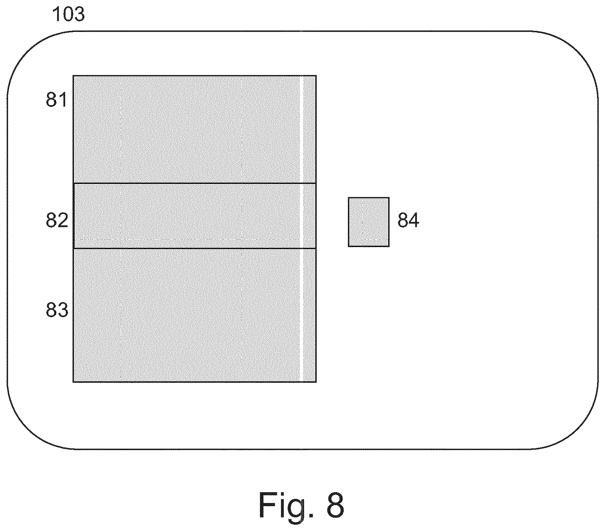

Fig. 8 illustrates a user interface presented on the computer screen 103. In this user interface, the user can follow the execution of the patch-clamp procedure. In window 81 previous process steps are shown, in window 82 the presently active process step is shown, and in window 83, the subsequent process steps are shown. The process steps are illustrated in the sequence intended for the selected patch-clamp procedure. The user interface allows the user to change this sequence by graphically dragging process steps to a new location in the row of process steps.

By a simple skip command, the user can skip a process step by the delete button 84 and jump to a subsequent process step.

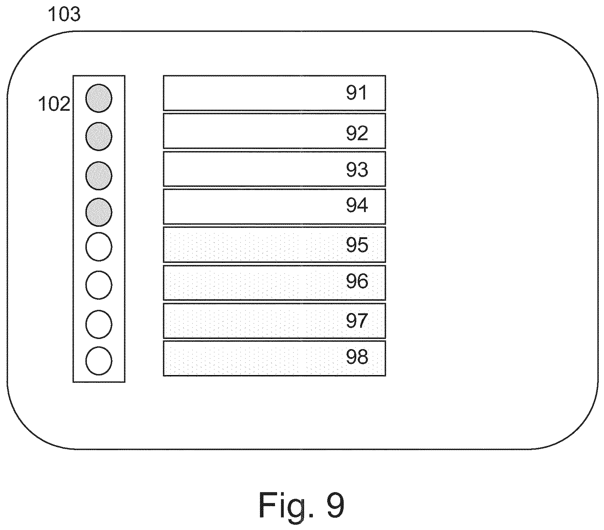

Fig. 9 illustrates a graphical user interface presented on the computer screen 103 instructing the user to add solutions to specific inlets of the array of inlets 102, in this case to the upper 4 of the 8 inlets. This provides easy and safe guidance for the user. The specific type of solution is specified in the windows 91-98.

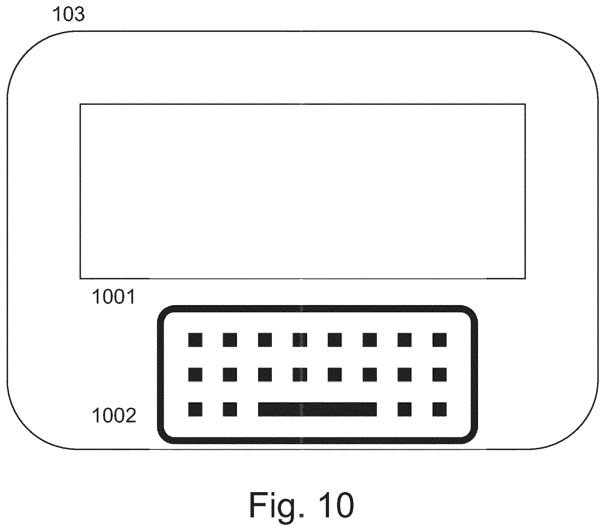

Fig. 10 illustrates a user interface presented on the computer screen 103 and allowing the user to key meta-data into window 1001 by use of the on-screen keyboard in window 1002. These meta-data are added to a data file and stored e.g. together with data defining the patch-clamp procedure which is presently selected.

## Claims

1. A semi-automated patch-clamp apparatus (100), comprising a patch-clamp plate manipulator configured to receive a patch-clamp plate (101) comprising a plurality of patch-clamp sites, and a computerised controller (103) configured to:
• define a patch-clamp procedure comprising process steps including manual process steps assigned to manual execution by a user and automatic process steps for automatic execution by the manipulator,
• provide instructions for a user to carry out the manual process steps, and
• provide commands for the manipulator for automatic execution of the automatic process steps;
wherein the controller is configured to execute the patch-clamp procedure by dispatching the commands to the manipulator for execution of the automatic process steps and by dispatching the instructions to the user for manual execution of the manual process steps, wherein the patch-clamp procedure comprises protocols each defining a sequence of signals applied automatically by the manipulator to the patch-clamp sites, and **characterised in that** the controller comprises a user interface allowing the user to define the protocols or to amend predefined protocols during execution of a patch-clamp procedure.

2. The apparatus according to claim 1, wherein the patch-clamp procedure comprises measurement steps defining readings made automatically by the manipulator at the patch-clamp sites.

3. The apparatus according to claim 2, wherein the controller comprises a user interface allowing the user to define the measurement steps or to amend predefined measurement steps.

4. The apparatus according to any of the preceding claims, wherein the controller comprises a user interface allowing the user to select predefined protocols and/or predefined measurement steps for a patch-clamp procedure.

5. The apparatus according to claim 3 or 4, wherein the user interface allows the user to define and/or select the measurement steps during execution of a patch-clamp procedure.

6. The apparatus according to any of the preceding claims, wherein the controller is configured to assign the manual process steps to a time critical group of manual process steps or to a non-time critical group of manual process steps, and wherein the instructions for a user to carry out steps assigned to manual execution is provided with:
• a timer countdown triggering a subsequent manual process step or protocol execution when the manual process step is assigned in the time critical group of manual process steps; and
• a user triggered acknowledgement triggering a subsequent manual process step or protocol execution when the manual process step is assigned in the non-time critical group of manual process steps.

7. The apparatus according to any of the preceding claims, wherein the controller is configured with a user interface allowing the user to re-assign a manual process step to an automatic process step, or to re-assign an automatic process step to a manual process step.

8. The apparatus according to any of the preceding claims, wherein the patch-clamp procedure comprises a sequence in which the process steps are executed, and the controller is configured to allow the user to skip a process step and jump to a subsequent process step in the sequence of process steps.

9. The apparatus according to any of the preceding claims, wherein the patch-clamp procedure comprises a sequence in which the process steps are executed, and the controller is configured to allow the user to change the sequence.

10. The apparatus according to any of the preceding claims, wherein the controller is configured to communicate results of a process step to the user before initiating a subsequent process step.

11. The apparatus according to any of the preceding claims, wherein the controller comprises a user interface allowing the user to add meta data related to the patch-clamp procedure, and wherein the controller is configured to define a data file comprising the meta data with data related to the patch-clamp procedure.

12. The apparatus according to claim 11, wherein the user interface allowing the user to add meta data related to the patch-clamp procedure, allows the user to add meta data individually to selected process steps of the patch-clamp procedure.

13. The apparatus according to claim 11 or 12, wherein the controller is configured to automatically add meta data to the patch-clamp procedure or to process steps of the patch-clamp procedure.

14. The apparatus according to claim 2 and 13, wherein the automatically added meta data relates to measurement steps.

15. A method of carrying out a patch-clamp procedure in a device according to any of the preceding claims, the method comprising predefining the procedure in the form of a plurality of manual process steps and a plurality of automatic process steps in a computer system and allowing the computer system to:
- trigger execution of the automatic process steps by electronically controlling a manipulator to handle the patch-clamp procedure including suppling solutions to patch-clamp sites, controlling pressure at the patch-clamp sites, and carrying out a procedure where an electrical and/or a pressure signal and/or an optical signal is applied to the patch-clamp sites and an electrical signal is received from the patch-clamp sites, and
- trigger the manual process steps by instructing a user, and further **characterised in that** the predefined protocols may be amended by the user during execution of a patch-clamp procedure.

## Patentansprüche

1. Halbautomatisierte Patch-Clamp-Vorrichtung (100), die einen Patch-Clamp-Plattenmanipulator, der zum Aufnehmen einer Patch-Clamp-Platte (101) konfiguriert ist, die eine Mehrzahl von Patch-Clamp-Orten umfasst, und einen computerisierten Regler (103) umfasst, der konfiguriert ist:
• eine Patch-Clamp-Vorgehensweise zu definieren, die Prozessschritte einschließlich manuelle Prozessschritte, die manueller Ausführung durch einen Benutzer zugewiesen sind, und automatische Prozessschritte für die automatische Ausführung durch den Manipulator umfasst,
• Anweisungen für einen Benutzer abzugeben, die manuellen Prozessschritte auszuführen und
• Befehle für den Manipulator zur automatischen Ausführung der automatischen Prozessschritte abzugeben;
wobei der Regler konfiguriert ist, die Patch-Clamp-Vorgehensweise durch Senden der Befehle an den Manipulator zur Ausführung der automatischen Prozessschritte und durch Senden der Anweisungen an den Benutzer zur manuellen Ausführung der manuellen Prozessschritte auszuführen, wobei die Patch-Clamp-Vorgehensweise Protokolle umfasst, von denen jedes eine Folge von Signalen definiert, die automatisch durch den Manipulator an den Patch-Clamp-Orten angewandt werden, und **dadurch gekennzeichnet, dass** der Regler
eine Benutzerschnittstelle umfasst, die dem Benutzer gestattet, die Protokolle zu definieren oder vordefinierte Protokolle während der Ausführung der Patch-Clamp-Vorgehensweise zu berichtigen.

2. Vorrichtung nach Anspruch 1, wobei die Patch-Clamp-Vorgehensweise Messschritte umfasst, die Ablesungen definieren, die automatisch durch den Manipulator an den Patch-Clamp-Orten gemacht werden.

3. Vorrichtung nach Anspruch 2, wobei der Regner eine Benutzerschnittstelle umfasst, die dem Benutzer gestattet, die Messschritte zu definieren oder vorbestimmte Messschritte zu berichtigen.

4. Vorrichtung nach einem der vorhergehenden Ansprüche, wobei der Regler eine Benutzerschnittstelle umfasst, die dem Benutzer gestattet, vordefinierte Protokolle und/oder vordefinierte Messschritte für eine Patch-Clamp-Vorgehensweise auszuwählen.

5. Vorrichtung nach Anspruch 3 oder 4, wobei die Benutzerschnittstelle dem Benutzer gestattet, die Messschritte während der Ausführung einer Patch-Clamp-Vorgehensweise zu definieren und/oder auszuwählen.

6. Vorrichtung nach einem der vorhergehenden Ansprüche, wobei der Regler konfiguriert ist, die manuellen Prozessschritte einer zeitkritischen Gruppe manueller Prozessschritte oder einer nichtzeitkritischen Gruppe manueller Prozessschritte zuzuweisen und wobei die Anweisungen für einen Benutzer, Schritte auszuführen, die der manuellen Ausführung zugewiesen sind, mit Folgendem versehen sind:
• einer Zeitmesserzeitkontrolle, die einen darauffolgenden manuellen Prozessschritt oder eine Protokollausführung auslöst, wenn der manuelle Prozessschritt der zeitkritischen Gruppe manueller Prozessschritte zugeordnet ist; und
• einer benutzerausgelöste Bestätigung, die einen darauffolgenden manuellen Prozessschritt oder eine Protokollausführung auslöst, wenn der manuelle Prozessschritt der nichtzeitkritischen Gruppe manueller Prozessschritte zugeordnet ist.

7. Vorrichtung nach einem der vorhergehenden Ansprüche, wobei der Regler mit einer Benutzerschnittstelle konfiguriert ist, die dem Benutzer gestattet, einen manuellen Prozessschritt einem automatischen Prozessschritt zuzuweisen oder einen automatischen Prozessschritt einem manuellen Prozessschritt zuzuweisen.

8. Vorrichtung nach einem der vorhergehenden Ansprüche, wobei die Patch-Clamp-Vorgehensweise eine Folge umfasst, in der die Prozessschritte ausgeführt werden und der Regler konfiguriert ist, dem Benutzer zu gestatten, einen Prozessschritt zu überspringen und zu einem darauffolgenden Prozessschritt in der Folge von Prozessschritten zu hüpfen.

9. Vorrichtung nach einem der vorhergehenden Ansprüche, wobei die Patch-Clamp-Vorgehensweise eine Folge umfasst, in der die Prozessschritte ausgeführt werden und der Regler konfiguriert ist, dem Benutzer zu gestatten, die Folge zu ändern.

10. Vorrichtung nach einem der vorhergehenden Ansprüche, wobei der Regler konfiguriert ist, Ergebnisse eines Prozessschritts dem Benutzer vor Beginn eines darauffolgenden Prozessschritts mitzuteilen.

11. Vorrichtung nach einem der vorhergehenden Ansprüche, wobei der Regler eine Benutzerschnittstelle umfasst, die dem Benutzer gestattet, Metadaten zuzufügen, die mit der Patch-Clamp-Vorgehensweise verbunden sind, und wobei der Regler konfiguriert ist, eine Datendatei zu definieren, die die Metadaten mit Daten umfasst, die mit der Patch-Clamp-Vorgehensweise verbunden sind.

12. Vorrichtung nach Anspruch 11, wobei die Benutzerschnittstelle, die dem Benutzer gestattet, Metadaten zuzufügen, die mit der Patch-Clamp-Vorgehensweise verbunden sind, dem Benutzer gestattet, Metadaten einzeln ausgewählten Prozessschritten der Patch-Clamp-Vorgehensweise zuzufügen.

13. Vorrichtung nach Anspruch 11 oder 12, wobei der Regler konfiguriert ist, automatisch Metadaten der Patch-Clamp-Vorgehensweise oder Prozessschritten der Patch-Clamp-Vorgehensweise zuzufügen.

14. Vorrichtung nach Anspruch 2 und 13, wobei die automatisch zugeführten Metadaten mit Messschritten verbunden sind.

15. Verfahren zum Ausführen einer Patch-Clamp-Vorgehensweise in einer Vorrichtung nach einem der vorhergehenden Ansprüche, wobei das Verfahren Vordefinieren der Vorgehensweise in Form einer Mehrzahl manueller Prozessschritte und einer Mehrzahl automatischer Prozessschritte in einem Computersystem und Gestatten dem Computersystem umfasst:
- Auslösen des Ausführens der automatischen Prozessschritte durch elektronisches Regulieren eines Manipulators zum Handhaben der Patch-Clamp-Vorgehensweise, einschließlich Liefern von Lösungen an Patch-Clamp-Orte, Regulieren des Drucks an Patch-Clamp-Orten und Ausführen einer Vorgehensweise, wobei ein elektrisches und/oder ein Drucksignal und/oder ein optisches Signal auf die Patch-Clamp-Orte aufgebracht wird und ein elektrisches Signal von den Patch-Clamp-Orten aufgenommen wird und
- Auslösen der manuellen Prozessschritte durch Anweisen eines Benutzers und ferner **dadurch gekennzeichnet, dass** die vordefinierten Protokolle durch den Benutzer während des Ausführens einer Patch-Clamp-Vorgehensweise geändert werden können.

## Revendications

1. Appareil de patch clamp semi-automatique (100), comprenant un manipulateur de plaque de patch clamp configuré pour recevoir une plaque de patch clamp (101) comprenant une pluralité de sites de patch clamp et un contrôleur informatisé (103) configuré pour :
• définir une procédure de patch clamp comprenant les étapes de traitement comprenant le traitement manuel les étapes assignées à l'exécution manuelle par un manipulateur et des étapes de traitement automatiques pour l'exécution automatique par le manipulateur,
• fournir des instructions pour un utilisateur pour réaliser les étapes de traitement manuel et
• fournir des commandes pour le manipulateur pour l'exécution automatique des étapes de traitement automatique ;
le contrôleur étant configuré pour exécuter la procédure de patch clamp en répartissant les commandes au manipulateur pour l'exécution des étapes de traitement automatique et en répartissant les instructions à l'utilisateur pour l'exécution manuelle des étapes de traitement manuel,
la procédure de patch clamp comprenant des protocoles chacun définissant une séquence de signaux appliquée automatiquement par le manipulateur aux sites de patch clamp et **caractérisé en ce que** le contrôleur comprend une interface d'utilisateur permettant à l'utilisateur de définir les protocoles ou d'amender des protocoles prédéfinis durant l'exécution d'une procédure de patch clamp.

2. Appareil selon la revendication 1, dans lequel la procédure de patch clamp comprend des étapes de mesure définissant des lectures faites automatiquement par le manipulateur aux sites de patch clamp.

3. Appareil selon la revendication 2, dans lequel le contrôleur comprend une interface d'utilisateur permettant à l'utilisateur de définir les étapes de mesure ou d'amender des étapes de mesure prédéfinies.

4. Appareil selon l'une quelconque des revendications précédentes, dans lequel le contrôleur comprend une interface d'utilisateur permettant à l'utilisateur de choisir des protocoles prédéfinis et/ou des étapes de mesure prédéfinies pour une procédure de patch clamp.

5. Appareil selon la revendication 3 ou 4, dans lequel l'interface d'utilisateur permet à l'utilisateur de définir et/ou de choisir les étapes de mesure durant l'exécution d'une procédure de patch clamp.

6. Appareil selon l'une quelconque des revendications précédentes, dans lequel le contrôleur est configuré pour assigner les étapes de traitement manuel à un groupe critique de temps d'étapes de traitement manuel ou à un groupe critique de non-temps d'étapes de traitement manuel et les instructions pour un utilisateur pour réaliser les étapes assignées à l'exécution manuelle étant fournies avec :
• un minuteur de décompte déclenchant une étape de traitement manuel subséquente ou l'exécution d'un protocole quand l'étape de traitement manuel est assignée dans le groupe critique de temps des étapes de traitement manuel ; et
• une reconnaissance déclenchée par l'utilisateur déclenchant une étape de traitement manuel subséquente ou l'exécution d'un protocole quand l'étape de traitement manuel est assignée dans le groupe critique de non-temps des étapes de traitement manuel.

7. Appareil selon l'une quelconque des revendications précédentes, dans lequel le contrôleur est configuré avec une interface d'utilisateur permettant à l'utilisateur de ré-assigner une étape de traitement manuel à une étape de traitement automatique ou de ré-assigner une étape de traitement automatique à une étape de traitement manuel.

8. Appareil selon l'une quelconque des revendications précédentes, dans lequel la procédure de patch clamp comprend une séquence dans laquelle les étapes de traitement sont exécutées et le contrôleur est configuré pour permettre à l'utilisateur de sauter une étape de traitement et de passer à une étape de traitement subséquente dans la séquence d'étapes de traitement.

9. Appareil selon l'une quelconque des revendications précédentes, dans lequel la procédure de patch clamp comprend une séquence dans laquelle les étapes de traitement sont exécutées et le contrôleur est configuré pour permettre à l'utilisateur de changer la séquence.

10. Appareil selon l'une quelconque des revendications précédentes, dans lequel le contrôleur est configuré pour communiquer les résultats d'une étape de traitement à l'utilisateur avant d'initier une étape de traitement subséquente.

11. Appareil selon l'une quelconque des revendications précédentes, dans lequel le contrôleur comprend une interface d'utilisateur permettant à l'utilisateur d'ajouter des méta données associées à la procédure de patch clamp et le contrôleur étant configuré pour définir un fichier de données comprenant les méta données avec les données associées à la procédure de patch clamp.

12. Appareil selon la revendication 11, dans lequel l'interface d'utilisateur permettant à l'utilisateur d'ajouter des méta données associées à la procédure de patch clamp, permet à l'utilisateur d'ajouter des méta données individuellement aux étapes de traitement choisies de la procédure de patch clamp.

13. Appareil selon la revendication 11 ou 12, dans lequel le contrôleur est configuré pour ajouter automatiquement des méta données à la procédure de patch clamp ou à des étapes de traitement de la procédure de patch clamp.

14. Appareil selon les revendications 2 et 13, dans lequel les méta données ajoutées automatiquement sont associées aux étapes de mesure.

15. Procédé de réalisation d'une procédure de patch clamp dans un dispositif selon l'une quelconque des revendications précédentes, le procédé comprenant la prédéfinition de la procédure sous la forme d'une pluralité d'étapes de traitement manuel et d'une pluralité d'étapes de traitement automatique dans un système informatique et permettant au système informatique de :
- déclencher l'exécution des étapes de traitement automatique en contrôlant électroniquement un manipulateur pour manipuler la procédure de patch clamp comprenant des solutions de fourniture à des sites de patch clamp, en contrôlant la pression aux sites de patch clamp et en réalisant une procédure ou un signal électrique et/ou de pression et/ou un signal optique est appliqué aux sites de patch clamp et un signal électrique est reçu des sites de patch clamp et
- déclencher les étapes de traitement manuel en instruisant un utilisateur et **caractérisé en outre en ce que** les protocoles prédéfinis peuvent être amendés par l'utilisateur durant l'exécution d'une procédure de patch clamp.
